(19) Europäisches Patentamt
European Patent Office
Office européen des brevets

(11) Veröffentlichungsnummer : **0 037 062**
**B1**

(12) **EUROPÄISCHE PATENTSCHRIFT**

(45) Veröffentlichungstag der Patentschrift : **17.10.84**

(21) Anmeldenummer : **81102228.4**

(22) Anmeldetag : **25.03.81**

(51) Int. Cl.[3] : **C 07 C 93/06**, C 07 C 97/07, A 61 K 31/135, C 07 C103/42, C 07 C 49/597, C 07 C 49/603, C 07 C 49/84

(54) **Substituierte 3-Aryl-2-cycloalken-1-one, Verfahren zu ihrer Herstellung und ihre Verwendung als Arzneimittel.**

(30) Priorität : **28.03.80 DE 3012012**

(43) Veröffentlichungstag der Anmeldung : **07.10.81 Patentblatt 81/40**

(45) Bekanntmachung des Hinweises auf die Patenterteilung : **17.10.84 Patentblatt 84/42**

(84) Benannte Vertragsstaaten : **AT BE CH DE FR GB IT LI NL SE**

(56) Entgegenhaltungen :
**DE-A- 2 210 842**
**DE-A- 2 636 725**
**GB-A- 1 215 751**
**E. Schröder e.a. Arzneimittelchemie Bd. II, Georg Thieme Verlag, Stuttgart, 1976**

**Die Akte enthält technische Angaben, die nach dem Eingang der Anmeldung eingereicht wurden und die nicht in dieser Patentschrift enthalten sind.**

(73) Patentinhaber : **Beiersdorf Aktiengesellschaft**
**Unnastrasse 48**
**D-2000 Hamburg 20 (DE)**

(72) Erfinder : **Cohnen, Erich, Dr. Dipl.-Chem.**
**Heilwigstrasse 25**
**D-2000 Hamburg 20 (DE)**

EP 0 037 062 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Gegenstand der Erfindung sind neue substituierte 3-Aryl-2-cycloalken-1-one der allgemeinen Formel I

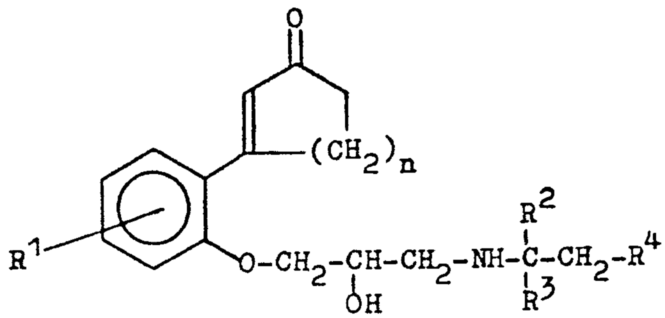

(I)

in der
$R^1$ ein Wasserstoff- oder Halogenatom, eine Alkyl- oder Acylaminogruppe mit jeweils 1 bis 4 Kohlenstoffatomen,
$R^2$ und $R^3$, die gleich oder verschieden sein können, ein Wasserstoffatom, eine Methyl- oder Äthylgruppe,
$R^4$ ein Wasserstoffatom oder eine Dimethoxyphenylgruppe und
n die Zahl 1 oder 2 bedeutet,
sowie deren physiologisch verträgliche Säureadditionssalze und Verfahren zu ihrer Herstellung und ihre Verwendung in pharmazeutischen Präparaten.

Ferner betrifft die Erfindung neue substituierte 3-Aryl-2-cycloalken-1-one der allgemeinen Formel II

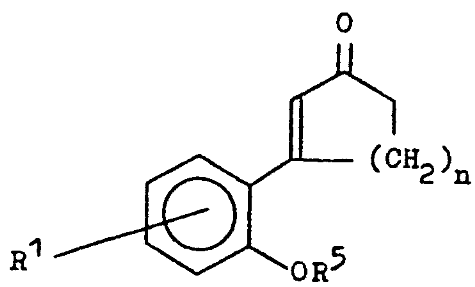

(II)

in der $R^1$ und n die oben angegebene Bedeutung haben und $R^5$ ein Wasserstoffatom, eine Benzylgruppe, den Rest

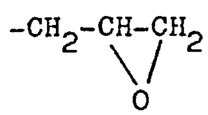

oder

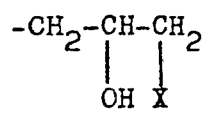

bedeutet, worin X für ein Chlor- oder Bromatom steht, und Verfahren zu ihrer Herstellung sowie ihre Verwendung als Zwischenprodukte für die Herstellung der erfindungsgemäßen Verbindungen der allgemeinen Formel I.

Die erfindungsgemäßen Verbindungen der allgemeinen Formel I und ihre Säureadditionssalze besitzen wertvolle therapeutische Eigenschaften. Sie zeichnen sich durch β-adrenolytische und blutdrucksenkende Wirkung aus und können daher zur Behandlung von Angina pectoris, Hypertonie und Arrhythmien eingesetzt werden. Die Dosierung beim Menschen beträgt 10 bis 200 mg pro Tag.

Es sind bereits β-adrenolytisch wirksame Verbindungen ähnlicher Struktur beschrieben worden (DE-A-2 636 725). Weiterhin ist bekannt (E. Schröder e. a., « Arzneimittelchemie » Bd. II, Georg Thieme Verlag, Stuttgart 1976), daß β-Blocker auch eine blutdrucksenkende Wirkung aufweisen können. Die erfindungsgemäßen Verbindungen zeichnen sich in überraschender Weise durch eine wesentlich höhere Wirkungsstärke aus.

Die Wirksamkeit der neuen Verbindungen ist unter anderem von der Stellung des Substituenten $R^1$ zur Propoxy-Seitenkette abhängig. Als besonders günstig haben sich solche Verbindungen erwiesen, in denen $R^1$ in meta-Stellung zu dieser Seitenkette steht. Weiterhin werden solche Verbindungen bevorzugt, die eine tert.-Butylaminogruppe als 3-Alkylaminogruppe der Propoxy-Seitenkette besitzen.

Die Verbindungen können als solche oder in Form ihrer Säureadditionssalze, gegebenenfalls mit geeigneten festen oder flüssigen pharmakologisch verträglichen Trägerstoffen und/oder Verdünnungsmitteln gebräuchlicher Art vermischt, zur Herstellung von Lösungen für Injektionszwecke und insbesondere von peroral zu verabreichenden pharmazeutischen Präparaten wie Dragees, Tabletten oder Liquida verwendet werden. Geeignete Trägerstoffe sind beispielsweise Milchzucker (Lactose), Gelatine, Maisstärke, Stearinsäure, Äthanol, Propylenglykol, Äther des Tetrahydrofurfurylalkohols und Wasser.

Das Verfahren zur Herstellung der Verbindungen der Formel I ist dadurch gekennzeichnet, daß man die neuen Verbindungen der Formeln III und IV

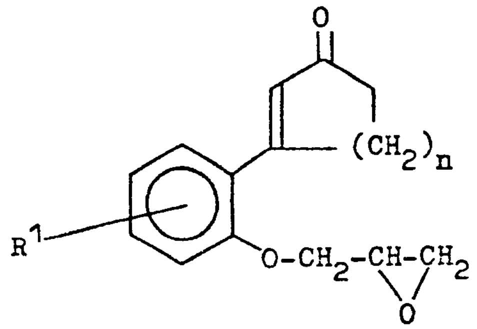

(III)

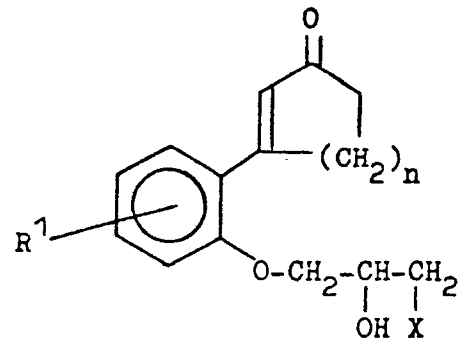

(IV)

worin $R^1$, n und X die obengenannte Bedeutung haben, mit einem Amin der allgemeinen Formel V

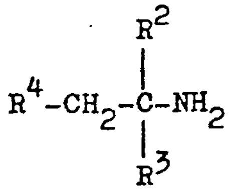

(V)

in der $R^2$, $R^3$ und $R^4$ die obengenannte Bedeutung haben, umsetzt. Die Reaktion wird in einem Alkohol, bevorzugt tert.-Butanol, mit einem Überschuß des Amins bei Temperaturen zwischen 20° und 60 °C durchgeführt.

Die erfindungsgemäßen freien Basen werden gegebenenfalls mit Säuren nach üblichen Verfahren in die physiologisch unbedenklichen Säureadditionssalze überführt. Geeignete Säuren sind beispielsweise Chlor- oder Bromwasserstoffsäure, Schwefel-, Oxal-, Fumar- oder Maleinsäure.

Als Ausgangsverbindungen zur Herstellung der Verbindungen III und IV sind die Verbindungen II geeignet, in denen $R^5$ ein Wasserstoffatom bedeutet. Diese Phenole sind erhältlich aus den entsprechenden Benzyläthern und lassen sich mit Epichlor- oder Epibromhydrin zu den Verbindungen der allgemeinen Formeln III und IV umsetzen, die als Gemische anfallen. Eine Trennung der Gemische ist durch Säulenchromatografie möglich, aber meist nicht erforderlich. Die Reaktion erfolgt mit einem Überschuß des Epihalogenhydrins entweder in Gegenwart einer katalytischen Menge einer organischen Base oder in Gegenwart eines säurebindenden Mittels, z. B. Natriumhydroxid oder Kaliumcarbonat, in geeigneten Lösungsmitteln, wie z. B. Alkoholen. Die Umsetzungen werden durch Erwärmen auf 50-100 °C beschleunigt.

Die Benzyläther gemäß der allgemeinen Formel II ($R^5$ = Benzyl) werden vorzugsweise durch Behandlung mit Bromwasserstoff/Eisessig-Gemischen bei Raumtemperatur gespalten.

Die Verbindungen der allgemeinen Formel II, in der $R^5$ eine Benzylgruppe bedeutet, können nach folgenden Verfahren hergestellt werden :

a) durch Grignard-Reaktion der Phenoläther der Formel VI

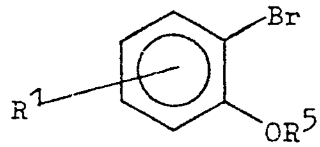

(VI)

in der $R^1$ die angegebene Bedeutung hat und $R^5$ die Benzylgruppe bedeutet, mit 3-Alkoxy-2-cycloalken-1-onen der allgemeinen Formel VII

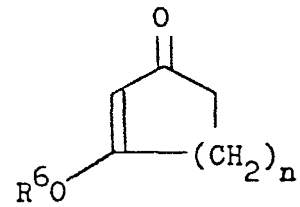

(VII)

wobei $R^6$ eine Methyl- oder Äthylgruppe und n die Zahl 1 oder 2 bedeutet. Die Reaktion wird in einem Äther, vorzugsweise Tetrahydrofuran, als Lösungsmittel bei Siedetemperatur durchgeführt. Die als Ausgangsmaterialien verwendeten Verbindungen der Formel VI sind entweder bekannte Verbindungen oder können nach Analogverfahren hergestellt werden. Die Alkoxycycloalkenone VII sind ebenfalls aus der Literatur bekannt.

b) durch alkalische Cyclisierung der 1-Phenyl-pentan-1,4-dione der Formel VIII

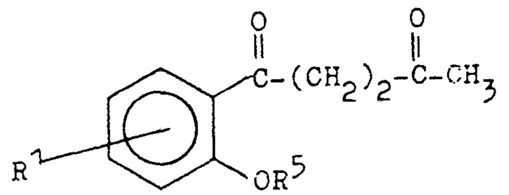

(VIII)

in der $R^1$ die oben angegebene Bedeutung hat und $R^5$ für eine Benzylgruppe steht. Auf diese Weise sind die erfindungsgemäßen Cyclopentenone erhältlich.

Verbindungen der Formel VIII erhält man aus den 2-Halogenacetophenonen der allgemeinen Formel IX

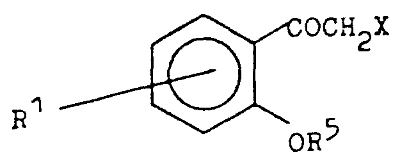

(IX)

worin $R^1$ die angegebene Bedeutung hat, $R^5$ die Benzylgruppe und X ein Chlor- oder Bromatom ist, indem man diese mit einem Alkalisalz des Acetessigsäuremethyl- oder -äthylesters zu den 1-Phenyl-3-carbalkoxy-pentan-1,4-dionen der allgemeinen Formel X

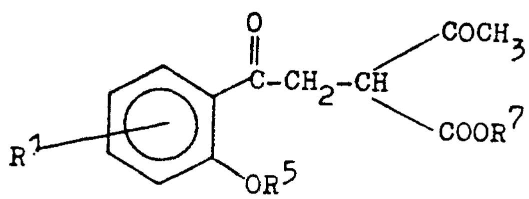

(X)

umsetzt, in der $R^1$ und $R^5$ die genannte Bedeutung haben und $R^7$ die Methylgruppe oder Äthylgruppe ist und diese durch Verseifung und Decarboxylierung mit Alkali in die 1,4-Diketone der Formel VIII überführt. Die Ausgangsverbindungen der Formel IX sind bekannt oder nach bekannten, in der Literatur beschriebenen Verfahren zugänglich.

Die folgenden Beispiele dienen zur Erläuterung der Erfindung :

## Beispiel 1

3-[2-(2-Hydroxy-3-tert.-butylamino-propoxy)-phenyl]-2-cyclopenten-1-on

2,0 g 3-(2-Hydroxyphenyl)-2-cyclopenten-1-on (Beispiel 9) werden mit 30 ml Epibromhydrin und katalytischen Mengen Piperidin 1 Stunde bei 100 °C gerührt. Nach Einengen wird das Gemisch aus Epoxi- und Bromhydrin-Verbindung durch Hochdruckflüssigchromatografie getrennt. Man erhält auf diese Weise 2 g 3-[2-(2,3-Epoxypropoxy)-phenyl]-2-cyclopenten-1-on als Öl, das in 20 ml tert.-Butanol gelöst wird und nach Zusatz von 2 ml tert.-Butylamin 48 Stunden bei Raumtemperatur gerührt wird.

Nach Abdampfen des Lösungsmittels wird der Rückstand in 2 n HCl aufgenommen und mit Methylenchlorid extrahiert. Die wäßrige Phase wird mit 2 n NaOH alkalisch gestellt und mit Methylenchlorid extrahiert. Die Extrakte werden getrocknet, mit äthanolischer HCl versetzt und eingedampft. Man löst den öligen Rückstand in heißem Äthanol und versetzt bis zur beginnenden Trübung mit Essigester. Das Hydrochlorid des 3-[2-(2-Hydroxy-3-tert.-butylamino-propoxy)-phenyl]-2-cyclopenten-1-on kristallisiert langsam aus.

Fp. 183-184 °C.

Analog Beispiel 1 werden die in Tabelle 1 aufgeführten Verbindungen dargestellt.

Tabelle 1

| Beisp. Nr. | $R^1$ | $R^2$ | $R^3$ | $R^4$ | n | Fp °C | Salz |
|---|---|---|---|---|---|---|---|
| 2 | H | $CH_3$ | H | H | 1 | 185-187 | Hydrochlorid |
| 3 | H | $CH_3$ | $CH_3$ | H | 2 | 186-187 | Hydrochlorid |
| 4 | 4-$CH_3$ | $CH_3$ | $CH_3$ | H | 1 | 198-203 | Hydrochlorid |
| 5 | 4-Cl | $CH_3$ | $CH_3$ | H | 1 | 218-222 | Hydrochlorid |
| 6 | 4-F | $CH_3$ | $CH_3$ | H | 1 | 222-226 | Hydrochlorid |
| 7 | 5-$CH_3$CONH- | $CH_3$ | $CH_3$ | H | 1 | 218-220 | Fumarat |
| 8 | H | H | H | 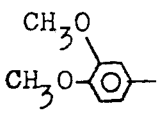 | 1 | 191-192(Z) | Hydrochlorid |

## Beispiel 9

3-(2-Hydroxyphenyl)-2-cyclopenten-1-on

a) 43,0 g (0,33 mol) Acetessigsäureäthylester werden unter Stickstoff zu einer Suspension von 7,9 g Natriumhydrid in 250 ml absolutem Toluol bei 50-60 °C getropft. Nach Beendigung der Wasserstoff-Entwicklung werden bei 40-60 °C 91,5 g (0,3 mol) 2-Benzyloxy-phenacylbromid in 400 ml Toluol zugetropft und anschließend 2 Stunden zum Sieden erhitzt. Nach Filtration des Natriumbromids wird die Toluollösung mehrfach mit Wasser gewaschen, über $MgSO_4$ getrocknet und eingedampft.

Man erhält ein Öl (100 g), das durch NMR-Spektroskopie als 1-(2-Benzyloxy-phenyl)-3-carbäthoxy-pentan-1,4-dion identifiziert wurde.

b) 85 g der Verbindung (a) werden mit 3 000 ml 2 %iger NaOH 2,5 Stunden unter Stickstoff auf 100 °C erhitzt. Nach Filtration eines Niederschlags extrahiert man mit Methylenchlorid, wäscht die organische Phase mit Wasser und trocknet über $MgSO_4$. Destillation des Methylenchlorid-Rückstands ($Kp._{0,2}$ 210-215 °C) ergibt ein Gemisch aus 65 % 1-(2-Benzyloxy-phenyl)-pentan-1,4-dion und 35 % 3-(Benzyloxy-phenyl)-2-cyclopenten-1-on.

36 g des Gemischs aus Diketon und Cyclopentenon werden mit 1,5 l 5 %iger NaOH 18 Stunden unter Stickstoff zum Sieden erhitzt. Man extrahiert mit Methylenchlorid und erhält nach üblicher Aufarbeitung und Umkristallisation aus Essigester/Äther 21,4 g 3-(2-Benzyloxy-phenyl)-2-cyclopenten-1-on.

Fp. 83-84 °C.

c) 8,0 g 3-(2-Benzyloxy-phenyl)-2-cyclopenten-1-on werden in 80 ml 20 %iger HBr-/Eisessig 1 Stunde bei Raumtemperatur gerührt. Nach Abdampfen des Eisessigs löst man den Rückstand in Methylenchlorid und neutralisiert durch Schütteln mit Natriumhydrogencarbonat-Lösung. Nach Umkristallisation aus Diisopropyläther/Diäthyläther erhält man 3,7 g 3-(2-Hydroxyphenyl)-2-cyclopenten-1-on.

Fp. 162-165 °C.

Analog Beispiel 9 wurden die folgenden in Tabelle 2 aufgeführten Verbindungen hergestellt :

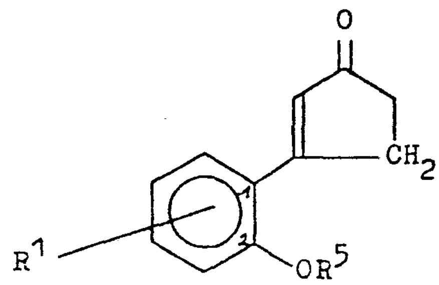

Tabelle 2

| Beispiel Nr. | $R^1$ | $R^5$ | Fp °C |
|---|---|---|---|
| 10 | $4-CH_3$ | $-CH_2C_6H_5$ | 131-132 |
| 11 | $4-CH_3$ | H | 187-189 |
| 12 | 4-Cl | $-CH_2C_6H_5$ | 94-95 |
| 13 | 4-Cl | H | 170-171 |
| 14 | 4-F | $-CH_2C_6H_5$ | 104-105 |
| 15 | 4-F | H | 157-159 |
| 16 | $5-NHCOCH_3$ | $-CH_2C_6H_5$ | 165-167 |
| 17 | $5-NHCOCH_3$ | H | 254-258 |

Beispiel 18

3-(2-Hydroxyphenyl)-2-cyclohexen-1-on

a) 32,9 g (0,125 mol) 2-Benzyloxy-brombenzol werden unter Rühren langsam zu 3,3 g (0,135 mol) Magnesiumspänen in 80 ml absolutem Tetrahydrofuran getropft. Anschließend wird 5 Stunden zum Sieden erhitzt. Die Lösung wird dann auf 20 °C gekühlt und man tropft innerhalb 30 Minuten 18,2 g (0,13 mol) 3-Äthoxy-2-cyclohexen-1-on bei 20-25 °C zu. Man erhitzt noch 1 Stunde zum Sieden, gießt auf ca. 500 ml gesättigte Ammonchlorid-Lösung und extrahiert mit Chloroform. Nach Umkristallisation aus Diisopropyläther erhält man 19,2 g 3-(2-Benzyloxyphenyl)-2-cyclohexen-1-on.

b) 11,0 g 3-(2-Benzyloxy-phenyl)-2-cyclohexen-1-on werden in 120 ml 20 %iger HBr/AcOH 1 Stunde bei Raumtemperatur gerührt. Nach Abdampfen des Eisessigs löst man den Rückstand in Methylenchlorid, neutralisiert mit $NaHCO_3$-Lösung und kristallisiert den $CH_2Cl_2$-Rückstand aus Diisopropyläther um.

Ausbeute : 5,3 g 3-(2-Hydroxyphenyl)-2-cyclohexen-1-on.

Fp. 126-128 °C.

Die Dosierung beim Menschen beträgt 5 bis 200 mg pro Tag bevorzugt sind 5 bis 50 mg pro Tag.

Die Tagesdosis ist individuell abzustimmen, weil sie von der Rezeptorempfindlichkeit und dem Sympathikotonus des Patienten abhängt. Zweckmäßigerweise wird die Behandlung mit niederen Dosen begonnen und dann gesteigert.

Zur Hypertoniebehandlung werden bevorzugt Dosen von 10 bis 20 mg einmal täglich verabreicht. Die Behandlung der Angina pectoris erfolgt vorzugsweise mit 5-10 mg einmal pro Tag. Zur Bekämpfung von Herzrhythmusstörungen werden 20-40 mg täglich, verteilt auf zwei Gaben, verabreicht.

Beispiel 19

Herstellung von Tabletten

Tabletten, welche die nachstehend angegebenen Bestandteile enthalten, werden nach bekannten

Arbeitsweisen hergestellt. Diese sind für die Behandlung von Hypertonie in einer Dosierungsmenge von 15 mg einmal täglich, für die Behandlung von Angina pectoris mit einer Menge von 10 mg einmal täglich und zur Behandlung von Arrhythmien mit einer Menge von zweimal 15 mg täglich geeignet.

| | Tablette A | Tablette B |
|---|---|---|
| 3-[2-(2-Hydroxy-3-tert.-butylamino-propoxy)-4-chlor-phenyl]-2-cyclo-penten-1-on-hydrochlorid (Beispiel 5) | 5 mg | 10 mg |
| Lactose | 89 mg | 84 mg |
| Maisstärke | 5 mg | 5 mg |
| Magnesiumstearat | 1 mg | 1 mg |

Beispiel 20

Herstellung von Tabletten

Tabletten, welche die nachstehend angegebenen Bestandteile enthalten, werden nach bekannten Arbeitsweise hergestellt. Diese sind für die Behandlung von Hypertonie in einer Dosierungsmenge von 15 mg einmal täglich, für die Behandlung von Angina pectoris mit einer Menge von 10 mg einmal täglich und zur Behandlung von Arrhythmien mit einer Menge von zweimal 15 mg täglich geeignet.

| | Tablette A | Tablette B |
|---|---|---|
| 3-[2-(2-Hydroxy-3-tert.-butylamino-propoxy)-4-fluor-phenyl]-2-cyclo-penten-1-on-hydrochlorid (Beispiel 6) | 5 mg | 10 mg |
| Lactose | 89 mg | 84 mg |
| Maisstärke | 5 mg | 5 mg |
| Magnesiumstearat | 1 mg | 1 mg |

**Ansprüche** (für die Vertragsstaaten : BE, CH, DE, FR, GB, IT, LI, NL, SE)

1. Substituierte 3-Aryl-2-cycloalken-1-one der allgemeinen Formel I

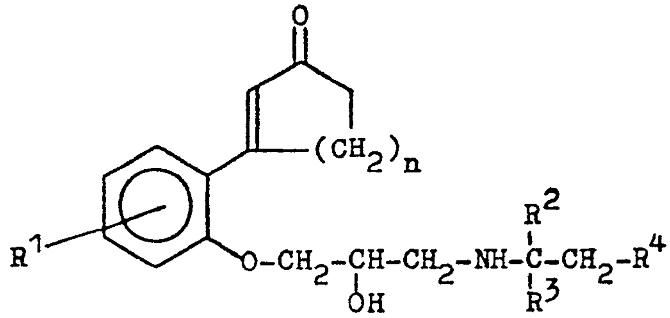

(I)

in der

$R^1$ ein Wasserstoff- oder Halogenatom, eine Alkyl- oder Acylaminogruppe mit jeweils 1 bis 4 Kohlenstoffatomen,

$R^2$ und $R^3$, die gleich oder verschieden sein können, ein Wasserstoffatom, eine Methyl- oder Äthylgruppe,

$R^4$ ein Wasserstoffatom oder eine Dimethoxyphenylgruppe und

n die Zahl 1 oder 2 bedeutet,

sowie deren physiologisch verträgliche Säureadditionssalze.

2. Substituierte 3-Aryl-2-cycloalken-1-one der allgemeinen Formel II

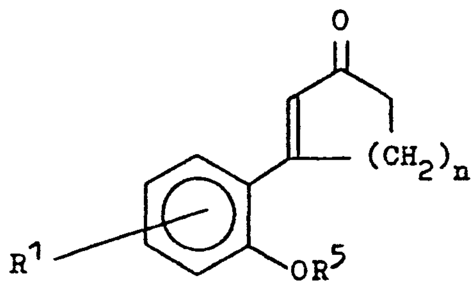

(II)

in der $R^1$ und n die oben angegebene Bedeutung haben und $R^5$ ein Wasserstoffatom, eine Benzylgruppe, den Rest

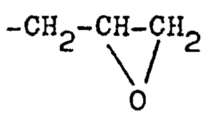 oder 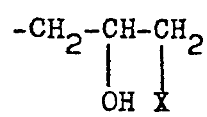

bedeutet, worin X für ein Chlor oder Bromatom steht.

3. Verfahren zur Herstellung der Verbindungen nach Anspruch 1 dadurch gekennzeichnet, daß man Verbindungen der Formel III und IV

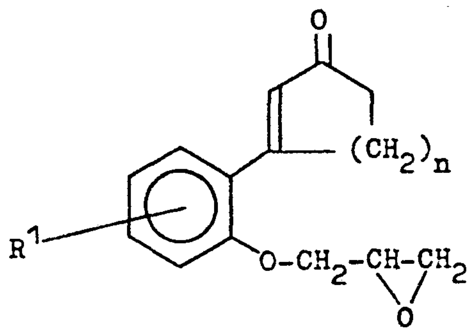

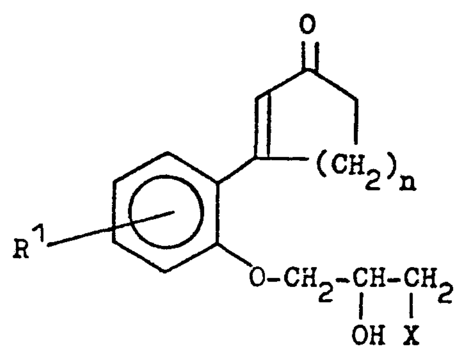

(III) (IV)

worin $R^1$, n und X die oben angegebene Bedeutung haben, oder ein Gemisch dieser Verbindungen, mit einem Amin der allgemeinen Formel V

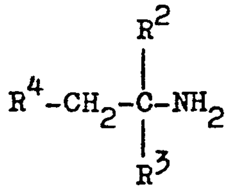

(V)

in der $R^2$, $R^3$ und $R^4$ die obengenannte Bedeutung haben, umsetzt, und gegebenenfalls die freien Basen mit Säuren in die entsprechenden Säureadditionssalze umwandelt.

4. Verfahren zur Herstellung der Verbindungen nach Anspruch 2, bei denen $R^5$ in der allgemeinen Formel II den Rest

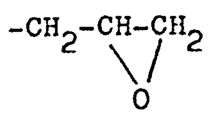

oder

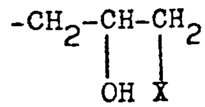

bedeutet und worin X für ein Chlor- oder Bromatom steht, dadurch gekennzeichnet, daß man eine Verbindung der allgemeinen Formel II, in der $R^5$ ein Wasserstoffatom bedeutet, mit Epichlor- oder Epibromhydrin umsetzt.

5. Verfahren zur Herstellung der Verbindungen nach Anspruch 2, bei denen $R^5$ in der allgemeinen Formel II ein Wasserstoffatom bedeutet, dadurch gekennzeichnet, daß man Verbindungen der allgemeinen Formel II, in denen $R^5$ eine Benzylgruppe bedeutet, einer säurekatalysierten Ätherspaltung unterwirft.

6. Verfahren zur Herstellung der Verbindungen nach Anspruch 2, bei denen $R^5$ in der allgemeinen Formel II eine Benzylgruppe bedeutet, dadurch gekennzeichnet, daß man Phenoläther der allgemeinen Formel VI

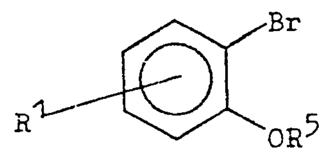

(VI)

in der $R^1$ die oben angegebene Bedeutung hat und $R^5$ die Benzylgruppe bedeutet, mit 3-Alkoxy-2-cycloalken-1-onen der allgemeinen Formel VII

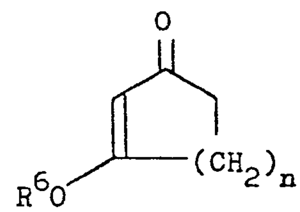

(VII)

in der n die Zahl 1 oder 2 und $R^6$ eine Methyl- oder Äthylgruppe bedeutet, in einer Grignard-Reaktion umsetzt.

7. Verfahren zur Herstellung der Verbindungen nach Anspruch 2, bei denen $R^5$ in der allgemeinen Formel II eine Benzylgruppe und n die Zahl 1 bedeutet, dadurch gekennzeichnet, daß man 1-Phenyl-pentan-1,4-dione der allgemeinen Formel VIII

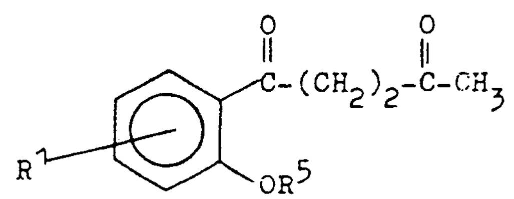

(VIII)

in der $R^1$ die oben angegebene Bedeutung hat und $R^5$ für die Benzylgruppe steht, einer alkalischen Cyclisierung unterwirft.

8. Verfahren zur Herstellung der Verbindungen der allgemeinen Formel VIII gemäß Anspruch 7, dadurch gekennzeichnet, daß man 2-Halogen-acetophenone der allgemeinen Formel IX

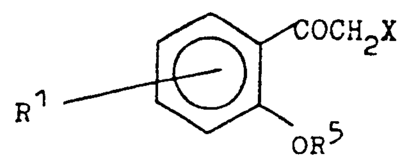

(IX)

in der $R^1$ die oben angegebene Bedeutung hat, $R^5$ die Benzylgruppe bedeutet und X ein Chlor- oder Bromatom ist, mit einem Alkalisalz des Acetessigsäuremethylesters oder -äthylesters zu den 1-Phenyl-3-carbalkoxy-pentan-1,4-dionen der allgemeinen Formel X

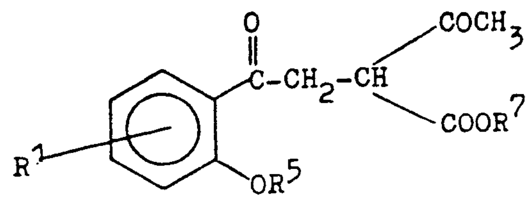

(X)

in der $R^1$ die oben angegebene Bedeutung hat und $R^5$ die Benzylgruppe und $R^7$ die Methylgruppe oder Äthylgruppe bedeutet, umsetzt, und diese durch Verseifung und Decarboxylierung in die Verbindungen der allgemeinen Formel VIII überführt.

9. Verwendung von Verbindungen nach Anspruch 2 als Zwischenprodukte für die Herstellung der Verbindungen der allgemeinen Formel I nach Anspruch 1.

10. Pharmazeutisches Präparat, dadurch gekennzeichnet, daß es eine oder mehrere der Verbindungen gemäß Anspruch 1 und gegebenenfalls übliche Trägerstoffe und/oder Verdünnungsmittel enthält.

11. Verbindungen der allgemeinen Formel I oder deren physiologisch verträgliche Säureadditionssalze gemäß Anspruch 1 zur Anwendung bei Angina pectoris, Hypertonie und Arrhytmien.

12. 3-[2-(2-Hydroxy-3-tert.-butylamino-propoxy)-4-chlor-phenyl]-2-cyclopenten-1-on.

13. 3-[2-(2-Hydroxy-3-tert.-butylamino-propoxy)-phenyl]-2-cyclopenten-1-on.

14. 3-[2-(2-Hydroxy-3-tert.-butylamino-propoxy)-4-methyl-phenyl]-2-cyclopenten-1-on.

15. 3-[2-(2-Hydroxy-3-tert.-butylamino-propoxy)-4-fluor-phenyl]-2-cyclopenten-1-on.

16. 3-[2-(2-Hydroxy-3-tert.-butylamino-propoxy)-5-acetamido-phenyl]-2-cyclopenten-1-on.

17. 3-[2-(2-Hydroxy-3-isopropylamino-propoxy)-phenyl]-2-cyclopenten-1-on.

18. 3-{2-[2-Hydroxy-3-(3,4-dimethoxy-phenyl)-äthylamino-propoxy]-phenyl}-2-cyclopenten-1-on.

19. 3-[2-(2-Hydroxy-3-tert.-butylamino-propoxy)-phenyl]-2-cyclohexen-1-on.

**Ansprüche** (für den Vertragsstaat AT)

1. Verfahren zur Herstellung von neuen substituierten 3-Aryl-2-cycloalken-1-onen der allgemeinen Formel I

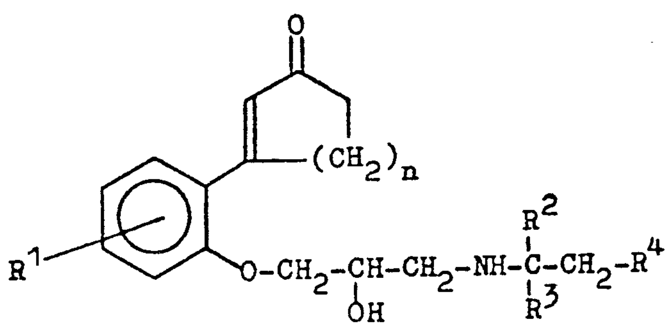

(I)

in der

$R^1$ ein Wasserstoff- oder Halogenatom, eine Alkyl- oder Acylaminogruppe mit jeweils 1 bis 4 Kohlenstoffatomen,

$R^2$ und $R^3$, die gleich oder verschieden sein können, ein Wasserstoffatom, eine Methyl- oder Äthylgruppe,

$R^4$ ein Wasserstoffatom oder eine Dimethoxyphenylgruppe und

n die Zahl 1 oder 2 bedeutet,

sowie deren physiologisch verträglichen Säureadditionssalzen, dadurch gekennzeichnet, daß man Verbindungen der Formeln III oder IV

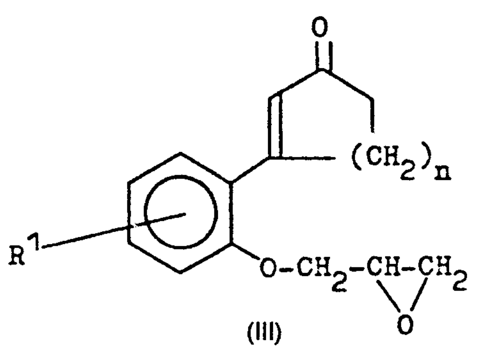

(III)

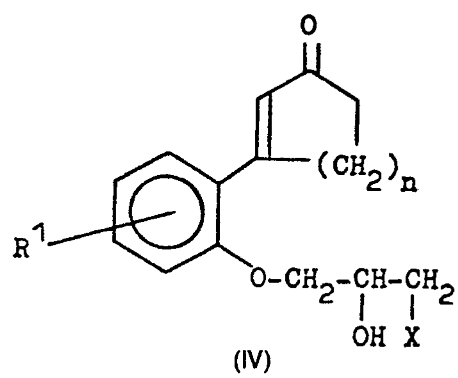

(IV)

worin $R^1$ und n die oben angegebenen Bedeutung haben und X Chlor oder Brom ist, oder ein Gemisch dieser Verbindungen, mit einem Amin der allgemeinen Formel V

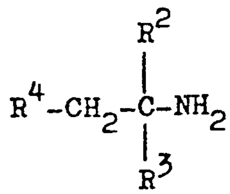

(V)

in der $R^2$, $R^3$ und $R^4$ die obengenannte Bedeutung haben, umsetzt, und gegebenenfalls die freien Basen mit Säuren in die entsprechenden Säureadditionssalze umwandelt.

2. Verfahren nach Anspruch 1 zur Herstellung von 3-[2-(2-Hydroxy-3-tert.-butylamino-propoxy)-4-chlorphenyl]-2-cyclopenten-1-on, dadurch gekennzeichnet, daß man 3-[2-(2,3-Epoxypropoxy)-4-chlorphenyl]-2-cyclopenten-1-on mit tert. Butylamin umsetzt.

3. Verfahren nach Anspruch 1 zur Herstellung von 3-[2-(2-Hydroxy-3-tert.-butylamino-propoxy)-phenyl]-2-cyclopenten-1-on, dadurch gekennzeichnet, daß man 3-[2-(2,3-Epoxypropoxy)-phenyl]-2-cyclopenten-1-on mit tert. Butylamin umsetzt.

4. Verfahren nach Anspruch 1 zur Herstellung von 3-[2-(2-Hydroxy-3-tert.-butylamino-propoxy)-4-methylphenyl]-2-cyclopenten-1-on, dadurch gekennzeichnet, daß man 3-[2-(2,3-Epoxypropoxy)-4-methylphenyl]-2-cyclopenten-1-on mit tert. Butylamin umsetzt.

5. Verfahren nach Anspruch 1 zur Herstellung von 3-[2-(2-Hydroxy-3-tert.-butylamino-propoxy)-4-fluorphenyl]-2-cyclopenten-1-on, dadurch gekennzeichnet, daß man 3-[2-(2,3-Epoxypropoxy)-4-fluorphenyl]-2-cyclopenten-1-on mit tert. Butylamin umsetzt.

6. Verfahren nach Anspruch 1 zur Herstellung von 3-[2-(2-hydroxy-3-tert.-butylamino-propoxy)-5-acetamidophenyl]-2-cyclopenten-1-on, dadurch gekennzeichnet, daß man 3-[2-(2,3-Epoxypropoxy)-5-acetamidophenyl]-2-cyclopenten-1-on mit tert. Butylamin umsetzt.

7. Verfahren nach Anspruch 1 zur Herstellung von 3-[2-(Hydroxy-3-isopropylamino-propoxy)-phenyl]-2-cyclopenten-1-on, dadurch gekennzeichnet, daß man 3-[2-(2,3-Epoxypropoxy)-phenyl]-2-cyclopenten-1-on mit Isopropylamin umsetzt.

8. Verfahren nach Anspruch 1 zur Herstellung von 3-{2-[2-Hydroxy-3-(2-(3,4-dimethoxy-phenyl)-äthylamino)-propoxy]-phenyl}-2-cyclopenten-1-on, dadurch gekennzeichnet, daß man 3-[2-(2,3-Expoxypropoxy)-phenyl]-2-cyclopenten-1-on mit 2-(3,4-Dimethoxyphenyl)-äthylamin umsetzt.

9. Verfahren nach Anspruch 1 zur Herstellung von 3-[2-(2-Hydroxy-3-tert.-butylamino-propoxy)-phenyl]-2-cyclohexen-1-on, dadurch gekennzeichnet, daß man 3-[2-(2,3-Epoxypropoxy)-phenyl]-2-cyclohexen-1-on mit tert. Butylamin umsetzt.

10. Verfahren zur Herstellung der neuen substituierten 3-Aryl-2-cycloalken-1-one der allgemeinen Formel II

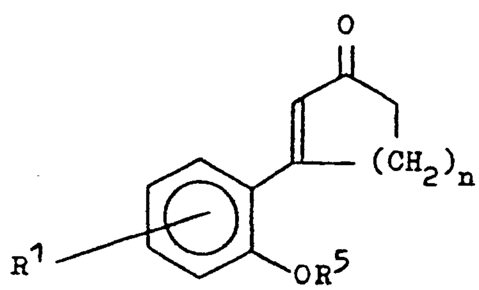

(II)

in der $R^1$ und n die in Anspruch 1 angegebenen Bedeutung haben und $R^5$ ein Wasserstoffatom, eine Benzylgruppe, den Rest

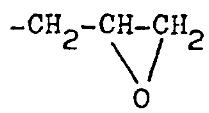

oder

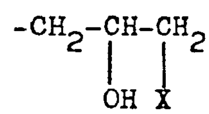

bedeutet, worin X für ein Chlor oder Bromatom steht, dadurch gekennzeichnet, daß man

a) zur Herstellung der Verbindungen der Formel II, in der $R^5$ den Rest

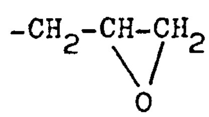

oder

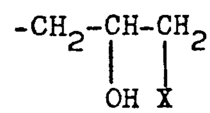

bedeutet, worin X für Chlor oder Brom steht, und $R^1$ und n die angegebene Bedeutung haben, eine Verbindung der allgemeinen Formel II, in der $R^5$ ein Wasserstoffatom bedeutet, mit Epichlor- oder Epibromhydrin umsetzt,

b) zur Herstellung der Verbindungen der Formel II in der $R^5$ ein Wasserstoffatom bedeutet und $R^1$ und n die angegebene Bedeutung haben, Verbindungen der allgemeinen Formel II, in denen $R^5$ eine Benzylgruppe bedeutet, einer säurekatalysierten Ätherspaltung unterwirft,

c) zur Herstellung der Verbindungen der allgemeinen Formel II, in der $R^5$ eine Benzylgruppe bedeutet, und $R^1$ und n die angegebene Bedeutung haben, Phenoläther der allgemeinen Formel VI

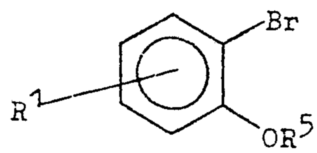

(VI)

in der $R^1$ die oben angegebene Bedeutung hat und $R^5$ die Benzylgruppe bedeutet, mit 3-Alkoxy-2-cycloalken-1-onen der allgemeinen Formel VII

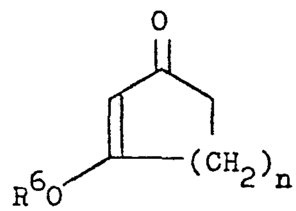

(VII)

in der n die Zahl 1 oder 2 und $R^6$ eine Methyl- oder Äthylgruppe bedeutet, in einer Grignard-Reaktion umsetzt, und

d) zur Herstellung der Verbindungen der Formel II in der $R^5$ eine Benzylgruppe und n die Zahl 1 bedeutet und $R^1$ die angegebene Bedeutung hat, 2-Halogen-acetophenone der allgemeinen Formel IX

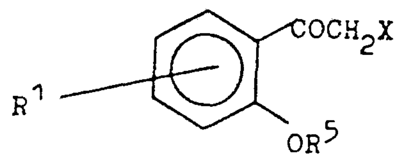

(IX)

in der $R^1$ die oben angegebene Bedeutung hat, $R^5$ die Benzylgruppe bedeutet und X ein Chlor- oder Bromatom ist, mit einem Alkalisalz des Acetessigsäuremethylesters oder -äthylesters zu den 1-Phenyl-3-carbalkoxy-pentan-1,4-dionen der allgemeinen Formel X

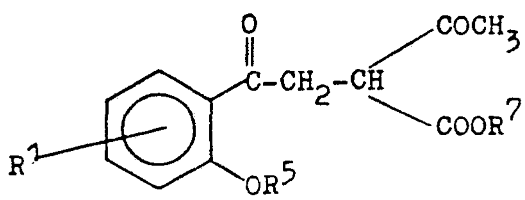

(X)

in der $R^1$ die oben angegebenen Bedeutung hat und $R^5$ die Benzylgruppe und $R^7$ die Methylgruppe oder Äthylgruppe bedeutet, umsetzt, und diese durch Verseifung und Decarboxylierung in die Verbindungen der allgemeinen Formel VIII überführt, und die so erhaltenen 1-Phenyl-pentan-1,4-dione der allgemeinen Formel VIII

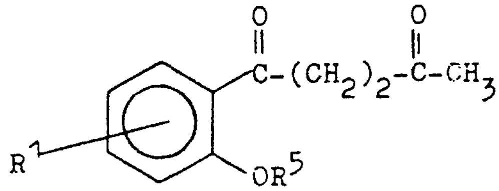

(VIII)

in der $R^1$ die oben angegebene Bedeutung hat und $R^5$ für die Benzylgruppe steht, einer alkalischen Cyclisierung unterwirft.

**Claims** (for the Contracting States : BE, CH, DE, FR, GB, IT, LI, NL, SE)

1. Substituted 3-aryl-2-cycloalken-1-ones of the general formula I

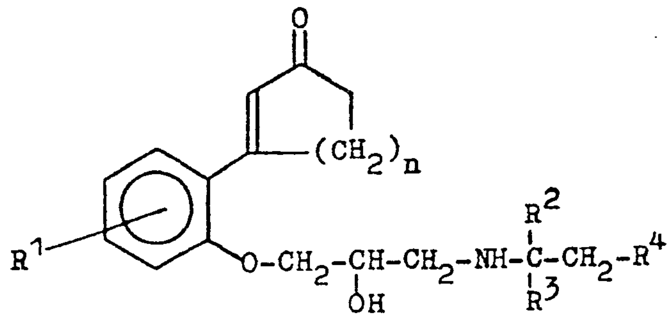

(I)

in which

$R^1$ denotes a hydrogen or halogen atom or an alkylamino or acylamino group having in each case 1 to 4 carbon atoms,

$R^2$ and $R^3$, which can be identical or different, denote a hydrogen atom or a methyl or ethyl group,

$R^4$ denotes a hydrogen atom or a dimethoxyphenyl group and

n denotes the number 1 or 2,

and also physiologically acceptable acid addition salts thereof.

2. Substituted 3-aryl-2-cycloalken-1-ones of the general formula II

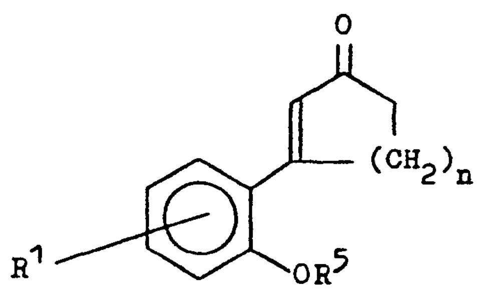

(II)

in which $R^1$ and n have the meaning indicated above and $R^5$ denotes a hydrogen atom, a benzyl group or the radical

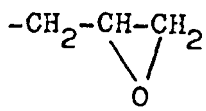

or

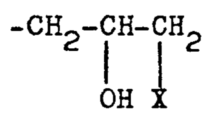

wherein X represents a chlorine or bromine atom.

3. Process for the preparation of the compounds according to Claim 1, characterised in that compounds of the formula III and IV

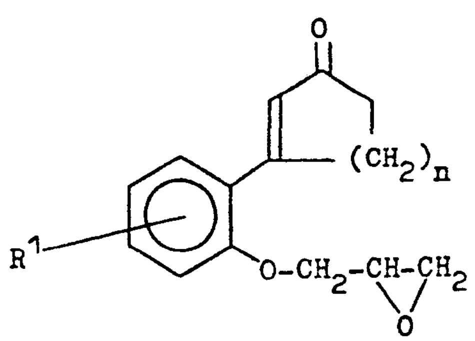

(III)

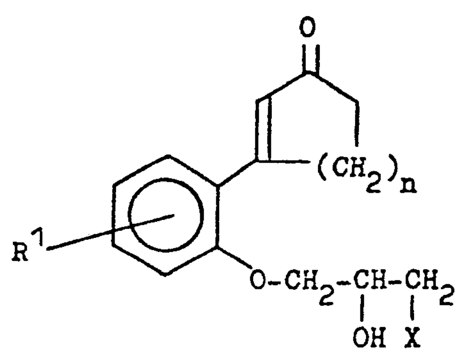

(IV)

wherein $R^1$, n and X have the meaning indicated above, or a mixture of these compounds, are reacted with an amine of the general formula V

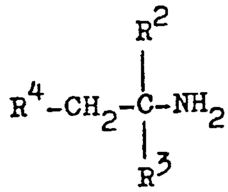

(V)

in which $R^2$, $R^3$ and $R^4$ have the abovementioned meanings, and, if appropriate, the free bases are converted into the corresponding acid addition salts by means of acids.

4. Process for the preparation of the compounds according to Claim 2 in which $R^5$ in the general formula II denotes the radical

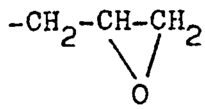 or 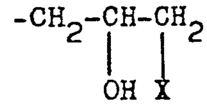

and wherein X represents a chlorine or bromine atom, characterised in that a compound of the general formula II in which $R^5$ denotes a hydrogen atom is reacted with epichlorohydrin or epibromohydrin.

5. Process for the preparation of the compounds according to Claim 2 in which $R^5$ in the general formula II denotes a hydrogen atom, characterised in that compounds of the general formula II in which $R^5$ denotes a benzyl group are subjected to an acid-catalysed ether cleavage.

6. Process for the preparation of the compounds according to Claim 2 in which $R^5$ in the general formula II denotes a benzyl group, characterised in that phenol ethers of the general formula VI

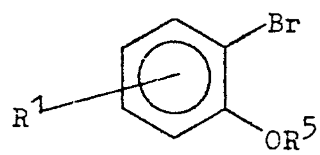

(VI)

in which $R^1$ has the meaning indicated above and $R^5$ denotes the benzyl group, are reacted in a Grignard reaction with 3-alkoxy-2-cycloalken-1-ones of the general formula VII

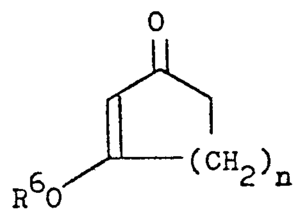

(VII)

in which n denotes the number 1 or 2 and $R^6$ denotes a methyl or ethyl group.

7. Process for the preparation of the compounds according to Claim 2 in which $R^5$ in the general formula II denotes a benzyl group and n denotes the number 1, characterised in that 1-phenylpentane-1,4-diones of the general formula VIII

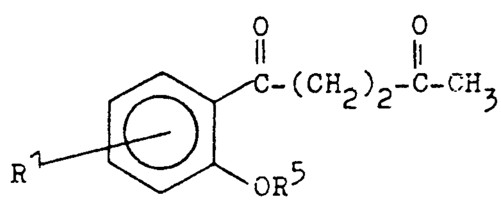

(VIII)

in which $R^1$ has the meaning indicated above and $R^5$ represents the benzyl group, are subjected to alkaline cyclisation.

8. Process for the preparation of the compounds of the general formula VIII according to Claim 7, characterised in that 2-halogenoacetophenones of the general formula IX

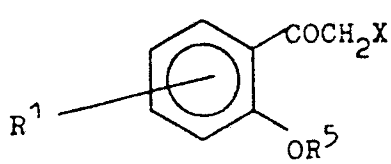

(IX)

in which $R^1$ has the meaning indicated above, $R^5$ denotes the benzyl group and X is a chlorine or bromine atom, are reacted with an alkali metal salt of methyl acetoacetate or ethyl acetoacetate to give the 1-phenyl-3-carboalkoxypentane-1,4-diones of the general formula X

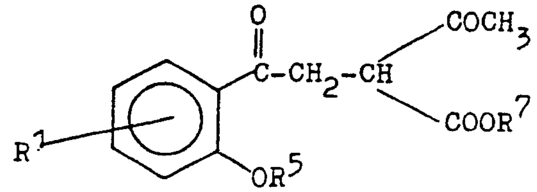

(X)

in which $R^1$ has the meaning indicated above and $R^5$ denotes the benzyl group and $R^7$ denotes the methyl or ethyl group, and the latter are converted into the compounds of the general formula VIII by saponification and decarboxylation.

9. The use of compounds according to Claim 2 as intermediate products for the preparation of the compounds of the general formula I according to Claim 1.

10. Pharmaceutical formulation characterised in that it contains one or more of the compounds according to Claim 1 and, if appropriate, customary excipients and/or diluents.

11. Compounds of the general formula I or physiologically acceptable acid addition salts thereof according to Claim 1 for use in Angina pectoris, hypertonia and arrhythmias.

12. 3-[2-(2-Hydroxy-3-tert.-butylaminopropoxy)-4-chlorophenyl]-2-cyclopenten-1-one.

13. 3-[-2-(2-Hydroxy-3-tert.-butylaminopropoxy)-phenyl]-2-cyclopenten-1-one.

14. 3-[2-(2-Hydroxy-3-tert.-butylaminopropoxy)-4-methylphenyl]-2-cyclopenten-1-one.

15. 3-[2-(2-Hydroxy-3-tert.-butylaminopropoxy)-4-fluorophenyl]-2-cyclopenten-1-one.

16. 3-[2-(2-Hydroxy-3-tert.-butylaminopropoxy)-5-acetamidophenyl]-2-cyclopenten-1-one.

17. 3-[2-(2-Hydroxy-3-isopropylaminopropoxy)-phenyl]-2-cyclopenten-1-one.

18. 3-{2-[2-Hydroxy-3-(3,4-dimethoxyphenyl)-ethylaminopropoxy]-phenyl}-2-cyclopenten-1-one.

19. 3-[2-(2-Hydroxy-3-tert.-butylaminopropoxy)-phenyl]-2-cyclohexen-1-one.

**Claims** (for the Contracting State AT)

1. Process for the preparation of new substituted 3-aryl-2-cycloalken-1-ones of the general formula I

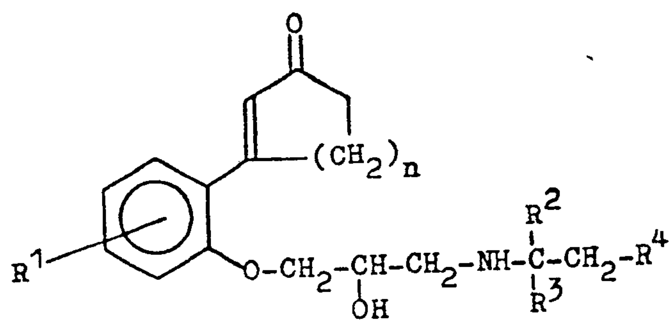

(I)

in which

$R^1$ denotes a hydrogen or halogen atom or an alkylamino or acylamino group having in each case 1 to 4 carbon atoms,

$R^2$ and $R^3$, which can be identical or different, denote a hydrogen atom or a methyl or ethyl group,

$R^4$ denotes a hydrogen atom or a dimethoxyphenyl group and

n denotes the number 1 or 2,

and also physiologically acceptable acid addition salts thereof, characterised in that compounds of the formulae III or IV

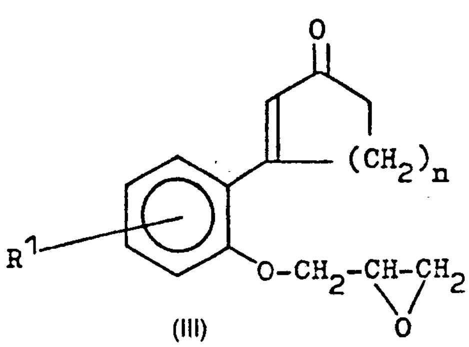

(III)

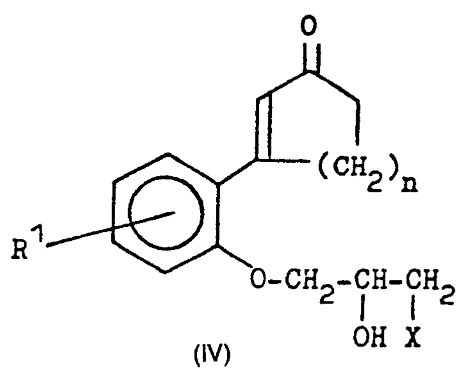

(IV)

wherein $R^1$ and n have the meaning mentioned above and X is chlorine or bromine, or a mixture of these compounds, are reacted with an amine of the general formula

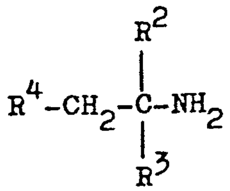

(V)

in which $R^2$, $R^3$ and $R^4$ have the abovementioned meaning, and, if appropriate, the free bases are converted into the corresponding acid addition salts by means of acids.

2. Process according to Claim 1 for the preparation of 3-[2-(2-hydroxy-3-tert.-butylaminopropoxy)-4-chlorophenyl]-2-cyclopenten-1-one, characterised in that 3-[2-(2,3-epoxypropoxy)-4-chlorophenyl]-2-cyclopenten-1-one is reacted with tert.-butylamine.

3. Process according to Claim 1 for the preparation of 3-[2-(2-hydroxy-3-tert.-butylaminopropoxy)-phenyl]-2-cyclopenten-1-one, characterised in that 3-[2-(2,3-epoxypropoxy)-phenyl]-2-cyclopenten-1-one is reacted with tert.-butylamine.

4. Process according to Claim 1 for the preparation of 3-[2-(2-hydroxy-3-tert.-butylaminopropoxy)-4-methylphenyl]-2-cyclopenten-1-one, characterised in that 3-[2-(2,3-epoxypropoxy)-4-methylphenyl]-2-cyclopenton-1-one is reacted with tert.-butylamine.

5. Process according to Claim 1 for the preparation of 3-[2-(2-hydroxy-3-tert.-butylaminopropoxy)-4-fluorophenyl]-2-cyclopenten-1-one, characterised in that 3-[2-(2,3-epoxypropoxy)-4-fluorophenyl]-2-cyclopenten-1-one is reacted with tert.-butylamine.

6. Process according to Claim 1 for the preparation of 3-[2-(2-hydroxy-3-tert.-butylaminopropoxy)-5-acetamidophenyl]-2-cyclopenten-1-one, characterised in that 3-[2-(2,3-epoxypropoxy)-5-acetamidophenyl]-2-cyclopenten-1-one is reacted with tert.-butylamine.

7. Process according to Claim 1 for the preparation of of 3-[2-(hydroxy-3-isopropylaminopropoxy)-phenyl]-2-cyclopenten-1-one, characterised in that 3-[2-(2,3-epoxypropoxy)-phenyl]-2-cyclopenten-1-one is reacted with isopropylamine.

8. Process according to Claim 1 for the preparation of 3-{2-[2-hydroxy-3-(2-(3,4-dimethoxyphenyl)-ethylamino)-propoxy]-phenyl}-2-cyclopenten-1-one, characterised in that 3-[2-(2,3-epoxypropoxy)-phenyl]-2-cyclopenten-1-one is reacted with 2-(3,4-dimethoxyphenyl)-ethylamine.

9. Process according to Claim 1 for the preparation of 3-[2-(2-hydroxy-3-tert.-butylaminopropoxy)-phenyl]-2-cyclohexen-1-one, characterised in that 3-[2-(2,3-epoxypropoxy)-phenyl]-2-cyclohexen-1-one is reacted with tert.-butylamine.

10. Process for the preparation of the new substituted 3-aryl-2-cycloalken-1-ones of the general formula II

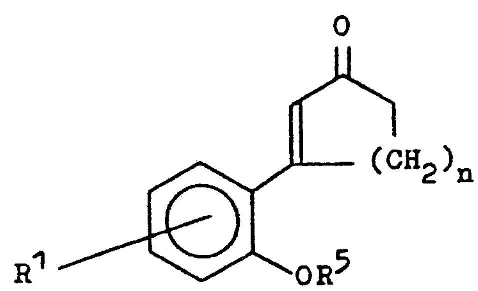

(II)

in which $R^1$ and n have the meaning indicated in Claim 1 and $R^5$ denotes a hydrogen atom, a benzyl group or the radical

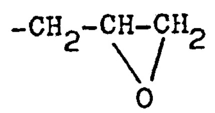 or 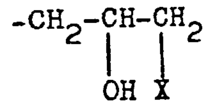

wherein X represents a chlorine or bromine atom, characterised in that

a) for the preparation of the compounds of the formula (II) in which $R^5$ denotes the radical

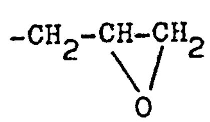 or 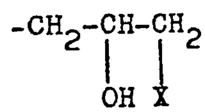

wherein X represents chlorine or bromine, and $R^1$ and n have the meaning indicated, a compound of the general formula (II) in which $R^5$ denotes a hydrogen atom, is reacted with epichlorohydrin or epibromohydrin,

b) for the preparation of the compounds of the formula II in which $R^5$ denotes a hydrogen atom and $R^1$ and n have the meaning indicated, compounds of the general formula II in which $R^5$ denotes a benzyl group, are subjected to an acid-catalysed ether cleavage,

c) for the preparation of the compounds of the general formula II in which $R^5$ denotes a benzyl group and $R^1$ and n have the meaning indicated, phenol ethers of the general formula VI

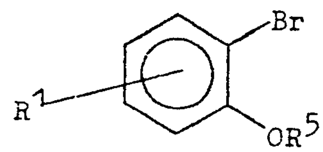

(VI)

in which $R^1$ has the meaning indicated above and $R^5$ denotes the benzyl group, are reacted in a Grignard reaction with 3-alkoxy-2-cycloalken-1-ones of the general formula VII

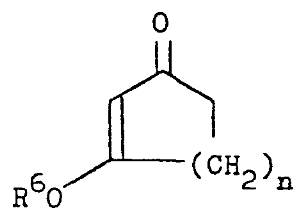

(VII)

in which n denotes the number 1 or 2 and $R^6$ denotes a methyl or ethyl group, and

d) for the preparation of the compounds of the formula II in which $R^5$ denotes a benzyl group and n denotes the number 1 and $R^1$ has the meaning indicated, 2-halogenoacetophenones of the general formula IX

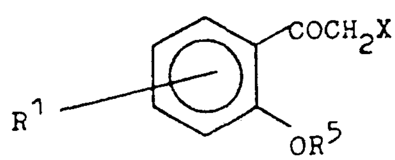

(IX)

in which $R^1$ has the meaning indicated above, $R^5$ denotes the benzyl group and X is a chlorine or bromine atom, are reacted with an alkali metal salt of methyl or ethyl acetoacetate to give the 1-phenyl-3-carboalkoxypentane-1,4-diones of the general formula X

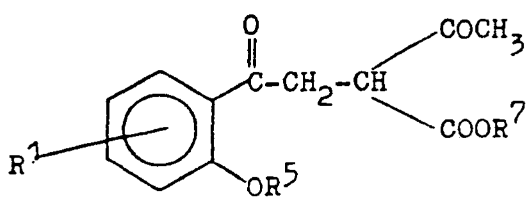

(X)

in which $R^1$ has the meaning indicated above and $R^5$ denotes the benzyl group and $R^7$ denotes the methyl or ethyl group, and the latter are converted by saponification and decarboxylation into the compounds of the general formula VIII, and the 1-phenylpentane-1,4-diones of the general formula VIII

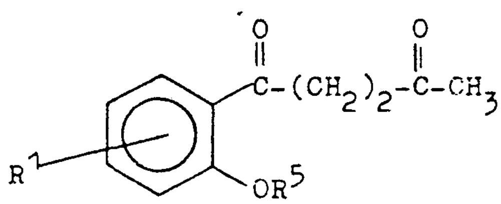

(VIII)

thus obtained in which $R^1$ has the meaning indicated above and $R^5$ represents the benzyl group, are subjected to alkaline cyclisation.

**Revendications** (pour les Etats contractants : BE, CH, DE, FR, GB, IT, LI, NL, SE)

1. 3-aryl-2-cycloalcén-1-ones substituées de formule générale I

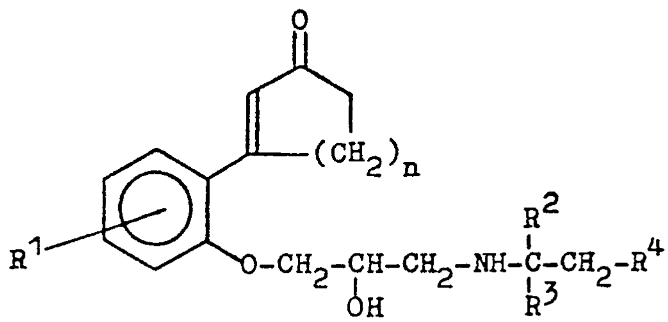

(I)

dans laquelle

$R^1$ représente un atome d'hydrogène ou un atome d'halogène, un groupe alkyl- ou acyl-amino contenant chacun 1 à 4 atomes de carbone,

$R^2$ et $R^3$, qui peuvent être identiques ou différents, représentent chacun un atome d'hydrogène, un groupe méthyle ou un groupe éthyle,

$R^4$ représente un atome d'hydrogène ou un groupe diméthoxyphényle et

n représente le nombre 1 ou 2,

ainsi que leurs sels d'addition d'acide physiologiquement compatibles.

2. 3-aryl-2-cycloalcén-1-ones substituées de formule générale II

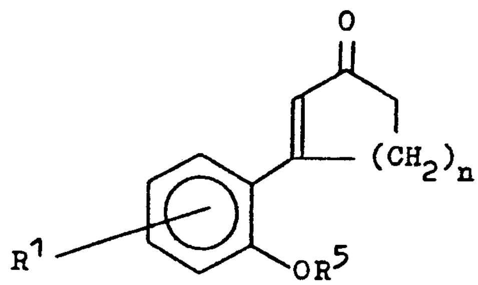

(II)

dans laquelle $R^1$ et n ont les significations indiquées ci-dessus et $R^5$ représente un atome d'hydrogène, un groupe benzyle, le radical

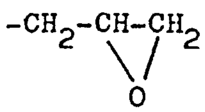

ou

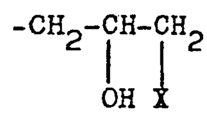

(III)

où X représente un atome de chlore ou un atome de brome.

3. Procédé de préparation des composés suivant la revendication 1, caractérisé en ce qu'on fait réagir des composés répondant aux formules III et IV

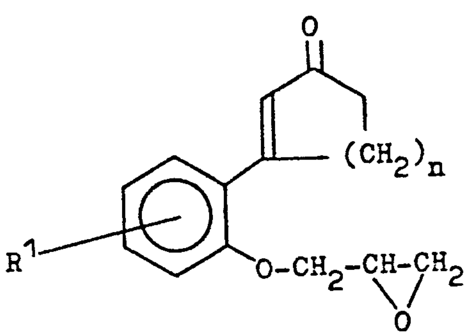

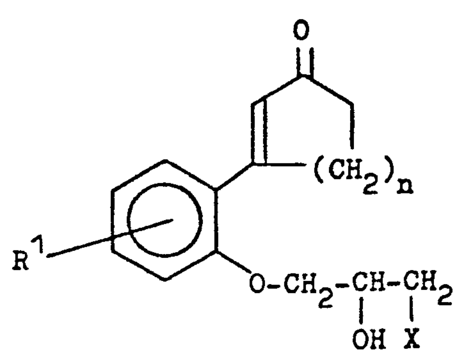

(IV)

(III)

(IV)

dans lesquelles $R^1$, n et X ont les significations indiquées ci-dessus, ou un mélange de ces composés avec une amine de formule générale V

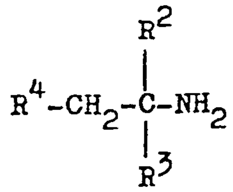

(V)

dans laquelle $R^2$, $R^3$ et $R^4$ ont les significations indiquées ci-dessus et on transforme éventuellement les bases libres en sels d'addition d'acide correspondants avec des acides.

4. Procédé de préparation des composés suivant la revendication 2 dans lesquels $R^5$ dans la formule générale II représente le radical

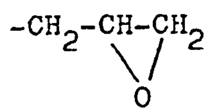

ou

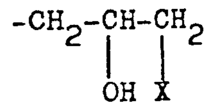

et où X représente un atome de chlore ou un atome de brome, caractérisé en ce qu'on fait réagir un composé de formule générale II dans laquelle $R^5$ représente un atome d'hydrogène, avec l'épichlorhydrine ou l'épibromhydrine.

5. Procédé de préparation des composés suivant la revendication 2 dans lesquels $R^5$ dans la formule générale II représente un atome d'hydrogène, caractérisé en ce qu'on soumet des composés de formule générale II dans lesquels $R^5$ représente un groupe benzyle, à une séparation d'éther catalysée par un acide.

6. Procédé de préparation des composés suivant la revendication 2 dans lesquels $R^5$ dans la formule générale II représente un groupe benzyle, caractérisé en ce qu'on fait réagir des phénol-éthers de formule générale VI

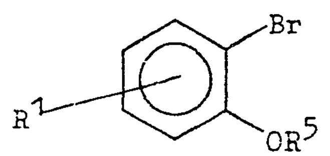

(VI)

dans laquelle $R^1$ a la signification indiquée ci-dessus et $R^5$ représente le groupe benzyle, avec des 3-alcoxy-2-cycloalcén-1-ones de formule générale VII

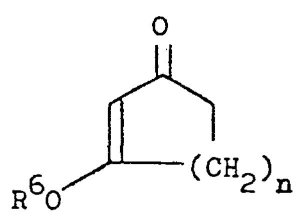

(VII)

dans laquelle n représente le nombre 1 ou 2 et $R^6$ représente un groupe méthyle ou un groupe éthyle, dans une réaction de Grignard.

7. Procédé de préparation des composés suivant la revendication 2 dans lesquels $R^5$ dans la formule générale II représente un groupe benzyle et n représente le nombre 1, caractérisé en ce qu'on soumet des 1-phényl-pentane-1,4-diones de formule générale VIII

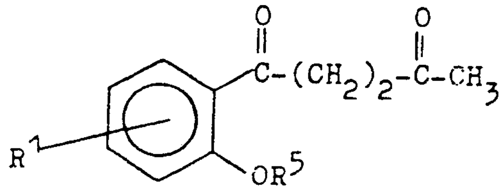

(VIII)

dans laquelle $R^1$ a la signification indiquée ci-dessus et $R^5$ représente le groupe benzyle, à une cyclisation alcaline.

8. Procédé de préparation des composés de formule générale VIII suivant la revendication 7, caractérisé en ce qu'on fait réagir des 2-halogénacétophénones de formule générale IX

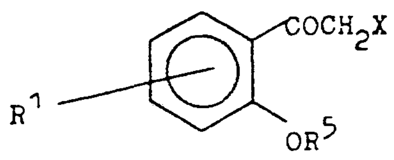

(IX)

dans laquelle $R^1$ a la signification indiquée ci-dessus, $R^5$ représente le groupe benzyle et X représente un atome de chlore ou un atome de brome, avec un sel alcalin de l'ester éthylique ou de l'ester méthylique d'acide acéto-acétique pour obtenir les 1-phényl-3-carbalcoxy-pentane-1,4-diones de formule générale X

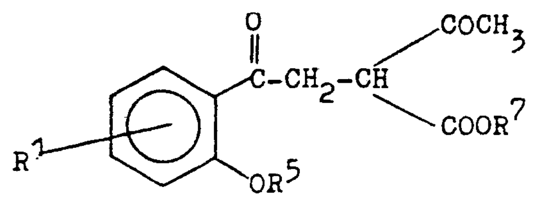

(X)

dans laquelle $R^1$ a la signification indiquée ci-dessus et $R^5$ représente le groupe benzyle, tandis que $R^7$ représente le groupe méthyle ou le groupe éthyle, et l'on transforme ces diones en composés de formule générale VIII par saponification et décarboxylation.

9. Utilisation des composés suivant la revendication 2 comme produits intermédiaires pour la préparation des composés de formule générale I suivant la revendication 1.

10. Préparation pharmaceutique, caractérisée en ce qu'elle contient un ou plusieurs des composés suivant la revendication 1 et éventuellement des substances supports et/ou des diluants habituels.

11. Composés de formule générale I ou leurs sels d'addition d'acide physiologiquement compatibles suivant la revendication 1 en vue de les utiliser pour l'angine de poitrine, l'hypertonie et les arhythmies.

12. La 3-[2-(2-hydroxy-3-tert-butylaminopropoxy)-4-chloro-phényl]-2-cyclopentén-1-one.

13. La 3-[2-(2-hydroxy-3-tert-butylaminopropoxy)-phényl]-2-cyclopentén-1-one.

14. La 3-[2-(2-hydroxy-3-tert-butylaminopropoxy)-4-méthyl-phényl]-2-cyclopentén-1-one.

15. La 3-[2-(2-hydroxy-3-tert-butylaminopropoxy)-4-fluoro-phényl]-2-cyclopentén-1-one.

16. La 3-[2-(2-hydroxy-3-tert-butylaminopropoxy)-5-acétamido-phényl]-2-cyclopentén-1-one.

17. La 3-[2-(2-hydroxy-3-isopropylaminopropoxy)-phényl]-2-cyclopentén-1-one.

18. La 3-{2-[2-hydroxy-3-(3,4-diméthoxyphényl)-éthylamino-propoxy]-phényl}-2-cyclopentén-1-one.

19. La 3-[2-(2-hydroxy-3-tert-butylaminopropoxy)-phényl]-2-cyclohexén-1-one.

**Revendications** (pour l'Etat contractant AT)

1. Procédé de préparation de nouvelles 3-aryl-2-cycloalcén-1-ones substituées de formule générale I

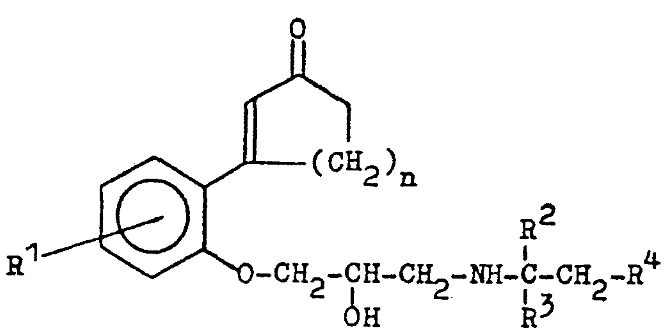

(I)

dans laquelle

$R^1$ représente un atome d'hydrogène ou un atome d'halogène, un groupe alkyl- ou acylamino contenant chacun 1 à 4 atomes de carbone,

$R^2$ et $R^3$, qui peuvent être identiques ou différents, représentent chacun un atome d'hydrogène, un groupe méthyle ou un groupe éthyle,

$R^4$ représente un atome d'hydrogène ou un groupe diméthoxyphényle, et

n représente le nombre 1 ou 2,

ainsi que de leurs sels d'addition d'acide physiologiquement compatibles, caractérisé en ce qu'on fait réagir des composés répondant aux formules III ou IV

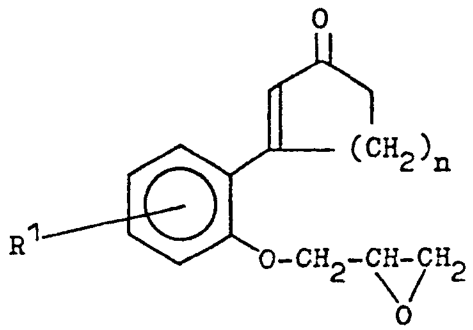

(III)

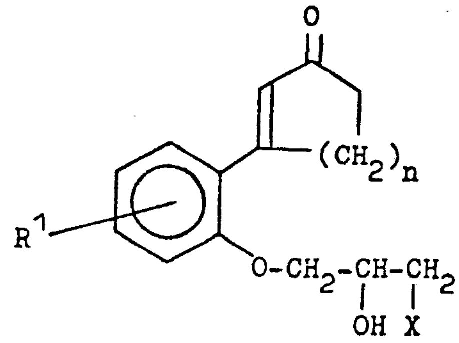

(IV)

dans lesquelles $R^1$ et n ont les significations indiquées ci-dessus et X représente un atome de chlore ou un atome de brome, ou un mélange de ces composés avec une amine de formule générale V

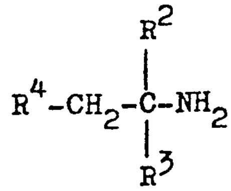

(V)

dans laquelle $R^2$, $R^3$ et $R^4$ ont les significations indiquées ci-dessus, et on transforme éventuellement les bases libres en sels d'addition d'acide correspondants, avec des acides.

2. Procédé suivant la revendication 1 pour la préparation de la 3-[2-(2-hydroxy-3-tert-butylaminopropoxy)-4-chlorophényl]-2-cyclopentén-1-one, caractérisé en ce qu'on fait réagir la 3-[2-(2,3-époxypropoxy)-4-chlorophényl]-2-cyclopentén-1-one avec la tert-butylamine.

3. Procédé suivant la revendication 1 pour la préparation de la 3-[2-(2-hydroxy-3-tert-butylaminopropoxy)-phényl]-2-cyclopentén-1-one, caractérisé en ce qu'on fait réagir la 3-[2-(2,3-époxypropoxy)-phényl]-2-cyclopentén-1-one avec la tert-butylamine.

4. Procédé suivant la revendication 1 pour la préparation de la 3-[2-(2-hydroxy-3-tert-butylaminopropoxy)-4-méthyl-phényl]-2-cyclopentén-1-one, caractérisé en ce qu'on fait réagir la 3-[2-(2,3-époxypropoxy)-4-méthyl-phényl]-2-cyclopentén-1-one avec la tert-butylamine.

5. Procédé suivant la revendication 1 pour la préparation de la 3-[2-(2-hydroxy-3-tert-butylaminopropoxy)-4-fluorophényl]-2-cyclopentén-1-one, caractérisé en ce qu'on fait réagir la 3-[2-(2,3-époxypropoxy)-4-fluorophényl]-2-cyclopentén-1-one avec la tert-butylamine.

6. Procédé suivant la revendication 1 pour la préparation de la 3-[2-(2-hydroxy-3-tert-butylaminopropoxy)-5-acétamidophényl]-2-cyclopentén-1-one, caractérisé en ce qu'on fait réagir la 3-[2-(2,3-époxypropoxy)-5-acétamidophényl]-2-cyclopentén-1-one avec la tert-butylamine.

7. Procédé suivant la revendication 1 pour la préparation de la 3-[2-(hydroxy-3-isopropylaminopropoxy)-phényl]-2-cyclopentén-1-one, caractérisé en ce qu'on fait réagir la 3-[2-(2,3-époxypropoxy)-phényl]-2-cyclopentén-1-one avec l'isopropylamine.

8. Procédé suivant la revendication 1 pour la préparation de la 3-{2-[2-hydroxy-3-(2-(3,4-diméthoxyphényl)-éthylamino)-propoxy]-phényl}-2-cyclopentén-1-one, caractérisé en ce qu'on fait réagir la 3-[2-(2,3-époxypropoxy)-phényl]-2-cyclopentén-1-one avec la 2-(3,4-diméthoxyphényl)-éthylamine.

9. Procédé suivant la revendication 1 pour la préparation de la 3-[2-(2-hydroxy-3-tert-butylaminopropoxy)-phényl]-2-cyclohexén-1-one, caractérisé en ce qu'on fait réagir la 3-[2-(2,3-époxypropoxy)-phényl]-2-cyclohexén-1-one avec la tert-butylamine.

10. Procédé de préparation des nouvelles 3-aryl-2-cycloalcén-1-ones substituées de formule générale II

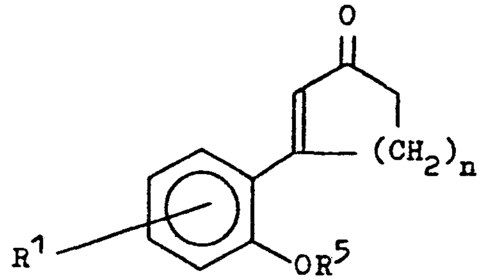

(II)

dans laquelle $R^1$ et n ont les significations indiquées dans la revendication 1 et $R^5$ représente un atome d'hydrogène, un groupe benzyle, le radical

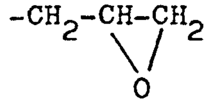 ou 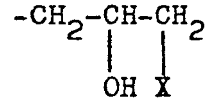

où X représente un atome de chlore ou un atome de brome, caractérisé en ce que

a) pour préparer les composés de formule II dans laquelle $R^5$ représente le radical

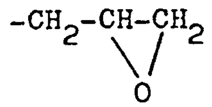 ou 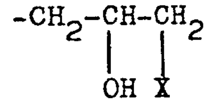

où X représente un atome de chlore ou un atome de brome, tandis que $R^1$ et n ont les significations indiquées, on fait réagir un composé de formule générale II dans laquelle $R^5$ représente un atome d'hydrogène, avec l'épichlorhydrine ou l'épibromhydrine,

b) pour préparer les composés de formule II dans laquelle $R^5$ représente un atome d'hydrogène, tandis que $R^1$ et n ont les significations indiquées, on soumet des composés de formule générale II dans lesquels $R^5$ représente un groupe benzyle, à une séparation d'éther catalysée par un acide,

c) pour préparer les composés de formule générale II dans laquelle $R^5$ représente un groupe benzyle, tandis que $R^1$ et n ont les significations indiquées, on fait réagir des phénoléthers de formule générale VI

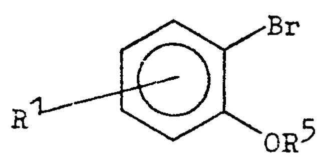

(VI)

dans laquelle $R^1$ a la signification indiquée ci-dessus et $R^5$ représente le groupe benzyle, avec des 3-alcoxy-2-cycloalcén-1-ones de formule générale VII

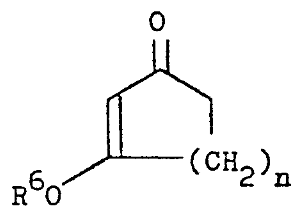

(VII)

dans laquelle n représente le nombre 1 ou 2 et $R^6$ représente un groupe méthyle ou un groupe éthyle, dans une réaction de Grignard, et

d) pour préparer les composés de formule II dans laquelle $R^5$ représente un groupe benzyle et n représente le nombre 1, tandis que $R^1$ a la signification indiquée, on fait réagir des 2-halogénacétophénones de formule générale IX

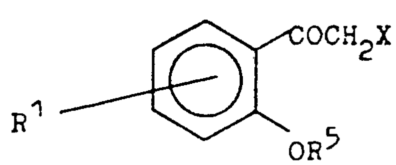

(IX)

dans laquelle $R^1$ a la signification indiquée ci-dessus, $R^5$ représente le groupe benzyle et X représente un atome de chlore ou un atome de brome, avec un sel alcalin de l'ester éthylique ou de l'ester méthylique d'acide acéto-acétique pour obtenir les 1-phényl-3-carbalcoxy-pentane-1,4-diones de formule générale X

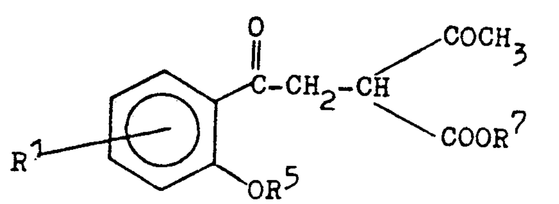

(X)

dans laquelle $R^1$ a la signification indiquée ci-dessus, $R^5$ représente le groupe benzyle et $R^7$ représente le groupe méthyle ou le groupe éthyle et l'on transforme ces diones en composés de formule générale VIII par saponification et décarboxylation, puis on soumet les 1-phényl-pentane-1,4-diones ainsi obtenues de formule générale VIII

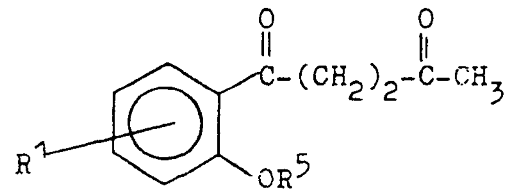

(VIII)

dans laquelle $R^1$ a la signification indiquée ci-dessus et $R^5$ représente le groupe benzyle, à une cyclisation alcaline.